# EUROPÄISCHE PATENTSCHRIFT

(11) **EP 0 386 202 B1**
(45) Veröffentlichungstag und Bekanntmachung des Hinweises auf die Patenterteilung: **12.05.1993**
(21) Anmeldenummer: 89909542.6
(22) Anmeldetag: 25.08.1989
(51) Int. Cl.: A47C 21/00, A47C 3/02

(54) **SITZ- ODER RUHEMÖBEL**
CHAIR OR BED
MEUBLE POUR S'ASSEOIR OU SE REPOSER

(30) Priorität: 28.08.1988 DE 8810986 U
(43) Veröffentlichungstag der Anmeldung: 12.09.1990
(73) Patentinhaber: Haider, Eduard, D-95704 Pullenreuth (DE)
(72) Erfinder: Haider, Eduard, D-95704 Pullenreuth (DE)
(74) Vertreter: Voigt, Günter, Dipl.-Ing.
(86) Internationale Anmeldenummer: DE8900565
(87) Internationale Veröffentlichungsnummer: WO9001886

(56) Entgegenhaltungen:
- DE-A- 3 506 377

## Beschreibung

Die Neuerung bezieht sich auf ein Sitz- oder Ruhemöbel gemäß Oberbegriff des Anspruchs 1.

Es hat bereits viele Versuche gegeben, die bisher weitverbreiteten statischen Sitz- und Ruhemöbel durch dynamische zu ersetzten.

So sind bereits seit langer Zeit Schaukelstühle bekannt, die anders als die üblichen Stühle eine dynamische Komponente aufweisen. Ganz Entsprechendes gilt auch für die ebenfalls seit langem bekannten Kinderwiegen, die wegen ihres dynamischen Charakters von den Kindern als besonders angenehm empfunden werden und sieh darüber hinaus als schlaffördernd erwiesen haben.

In jüngerer Zeit ist das Wasserbett als moderne Art eines dynamischen Bettes auf dem Markt erschienen. Das Wasserbett hat jedoch erhebliche Nachteile. indem es relativ viel Platz erfordert, träge auf Bewegungen reagiert, sehr schwer und recht teuer ist, sowie bei einem etwaigen Auslaufen des Wassers nicht unerhebliche Schäden verursacht. Darüber hinaus bietet das Wasserbett keine kontinuierlichen Auf- und Abbewegungen und kann auch keine stets gleichbleibend horizontale Liegefläche gewährleisten.

Aus der CH-PS 242 273 ist eine Sitzgelegenheit bekannt, die auf Pendelstützen gelagert ist und sieh in einem labilen Gleichgewicht befindet, aus dem sie bei Belastung mehr oder weniger ausgelenkt wird.

Aus dem DE-GM 1 708 191 ist eine an einem Schaukelgestell aufgehängte Sitzgelegenheit bekannt, die im Bedarfsfall auch arretiert werden kann. Ein solches Schaukelgestell ist für die Aufstellung in einem Schlaf- oder Wohnzimmer jedoch nicht geeignet.

Aus der GB-PS 696 239 ist ferner ein Schaukelstuhl bekannt, der über Gelenkpendel in gewissem Umfang nach vorn und rückwärts verschoben werden kann. Wegen der in den Gelenkpunkten auftretenden Reibung erfordert seine Verstellung jedoch nicht zu vernachlässigende Kräfte. Ein etwaiges Nachschwingen ist praktisch ausgeschlossen.

Aus dem DE-GM 8 222 691 ist eine Aufhängung an Seilpendeln bekannt, bei der nur noch eine vernachlässigbar geringe Bedämpfung der Pendelbewegungen stattfindet. Bei dieser bekannten Lösung gibt es jedoch nur eine Eigenfrequenz des schwingenden Systems, die sich aus der schwingenden Masse und der Pendellänge ergibt.

Aus der gattungsbildenden DE-PS 3 506 377 ist ein pendelnd aufgehängtes Sitz- oder Ruhemöbel bekannt, das mehrere funktionell in Reihe geschaltete Pendel unterschiedlicher Länge aufweist und somit verschiedene, einander benachbarte Eigenfrequenzen aufweist.

Der Neuerung liegt die Aufgabe zugrunde ein dynamisches Sitz- oder Ruhemöbel zu schaffen, das bereits bei geringsten Impulsen eine nahezu ungedämpfte Bewegung nicht unerheblicher Amplitude ermöglicht.

Die Lösung dieser Aufgabe erfolgt neuerungsgemäß mit Hilfe der Merkmale des Anspruchs 1.

Vorteilhafte Weiterbildungen ergeben sich aus den Unteransprüchen.

Die Lösung hat den erheblichen Vorteil, daß selbst geringe Impulse zu relativ großen Schwingungsausschlägen führen. Die gewünschte Wirkung wird damit weiter verbessert.

Die Neuerung wird naehfolgend unter Bezugnahme auf die Zeichnungsfiguren erläutert. Es zeigen:
- Fig. 1: eine schematisierte Darstellung der Pendelaufhängung,
- Fig. 2: eine Pendeleinrichtung gemäß Figur 1 mit die Seilpendel umgebenden Schmiegungstrichtern,
- Fig. 3: eine Draufsicht auf eine Pendeleinrichtung mit Verstellscheibe und
- Fig. 4: eine Seitenansicht einer Pendeleinrichtung mit Verstellscheibe.

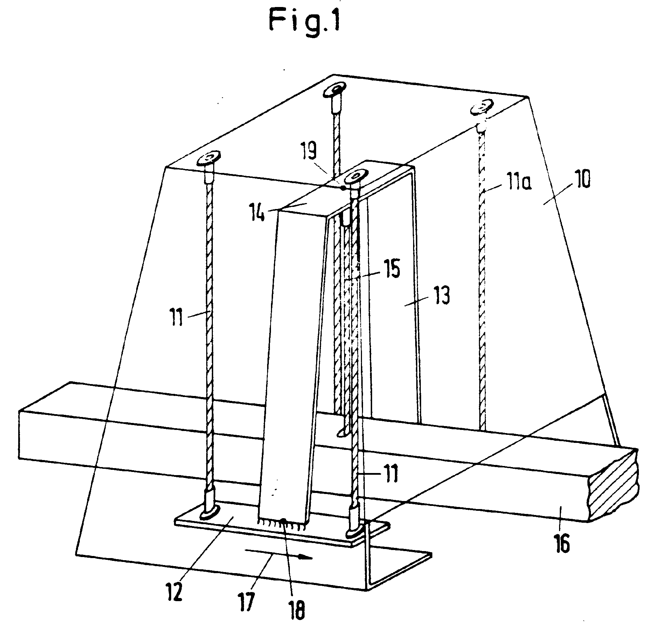

In Fig. 1 sind an einem Gehäuse 10, das im dargestellten Ausführungsbeispiel etwa pyramidenstumpfförmige Gestalt aufweist und auf einem festen Untergrund - beispielsweise auf dem Fußboden - steht, insgesamt vier Seilpendel 11, 11 a angeordnet, die jeweils paarweise eine Brücke 12 pendelnd tragen und mit dieser Brücke zusammen ein Trapez derart bilden, daß wahlweise ein spitzer oder ein stumpfer Trapezwinkel entsteht. Spiegelbildlich zu der Anordnung aus Seilpendeln 11 und Brücke 12 sind im Gehäuse 10 weitere Seilpendel 11a mit zugehöriger Brücke vorhanden, die ein zweites Trapez mit gleichen Trapezwinkeln bilden. Die beiden Trapeze 11, 12 bzw. 11a, 12, sind über ein im wesentlichen galgenförmiges Gerüst 13, das ebenfalls die Form eines Trapezes haben kann, fest miteinander verbunden. Am oberen Querbalken 14 des galgenförmigen Gerüstes 13 ist mindestens ein weiteres Seilpendel 15 aufgehängt, daß an seinem unteren Ende einen Träger 16 des beweglichen Oberteils des Sitz- oder Ruhemöbels trägt. Bei einer Schwingbewegung der beiden Trapeze 11, 12 bzw. 11a, 12, in Richtung des Pfeiles 17 führt die Brücke 12 gleichzeitig eine !eichte Nickbewegung im Uhrzeigersinn aus. Der Fußpunkt 18 des Gerüstes 13 wird dadurch geringfügig angehöben. Da gleichzeitig das mit der Brücke 12 verbundene Gerüst 13 ebenfalls eine leichte Nickbewegung im Uhrzeigersinn ausführt, verringert sich die Höhendifferenz zwischen dem Fußpunkt 18 und dem Aufhängepunkt 19 des Seilpendels 15 am Gerüst 13. Der am Seilpendel 15 aufgehängte Träger 16 führt somit lediglich eine verringerte Höhenverschiebung aus. Dies hat zur Folge, daß die bei einer Pendelbewegung auftretende Umwandlung von kinetischer Energie in potentielle Energie nur vermindert eintritt, weshalb auch die Abbremsung der Bewegung durch Energieumwandlung geringer als im Normalfall ist. Daraus ergeben sich erhöhte Schwingungsamplituden, was im vorliegenden Fall durchaus erwünscht ist.

Durch unterschiedliche Längen der Pendel 11 bzw. 11a einerseits und 15 andererseits ergeben sich Pendelsysteme mit untersehiedlichen Eigenfrequenzen, wodurch sichergestellt ist, daß das System nicht nur auf eine einzige Eigenfrequenz eingestimmt ist.

Das System paßt sich automatisch der jeweiligen Eigenfrequenz des Benutzers an (0,5 bis 3,5 Hz), beispielsweise der Herz- oder Atemfrequenz bzw. der freien Körperschwingung.

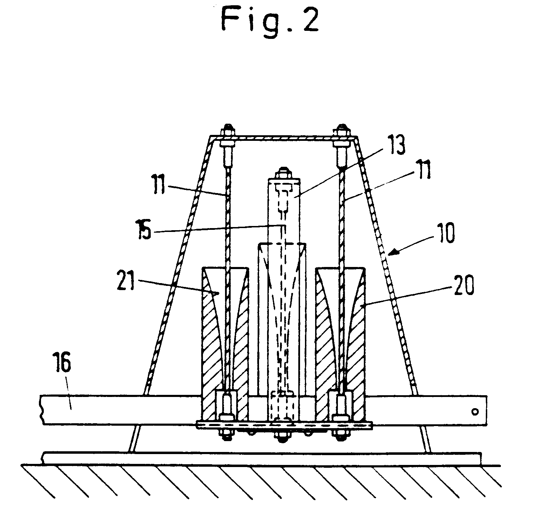

In Fig. 2 ist ein Gestell 10 mit Seilpendeln 11, einem galgenförmigen Gerüst 13 und einem Seilpendel 15 im in Richtung des Trägers 16 in geschnittenem Zustand dargestellt, bei dem die einzelnen Seilpendel 11 bzw. 15 mit Schmiegungstrichtern 21 umgeben sind, deren innerer lichter Querschnitt sich nach unten verjüngt. Der lichte Querschnitt kann dabei sowohl kreisringförmige als auch ovale Gestalt haben. Bei einer kreisringförmigen Gestalt ist das Verhalten in allen Richtungen gleich, bei einem ovalen Querschnitt ist jedoch das Verhalten in Richtung der Längsachse anders.als in Richtung der Querachse. Je nachdem, ob in allen Richtungen ein gleichmäßiges oder ein unterschiedliches Verhalten gewünscht wird, kann der eine oder andere lichte Querschnitt der Schmiegungstrichter 21 gewählt werden. Bei Amplituden, die ein gewisses Maß überschreiten, legen sich die Seilpendel 11 bzw. 15 an den lichten Querschnitt des Schmiegungstrichters 21 bis zu einer bestimmten Höhe an und verringern damit die wirksame Seillänge und so auch die Eigenfrequenz des Seilpendels 11 bzw. 15 derart, daß ein weiteres Ansteigen der Ampitude sanft und ruckfrei vermieden wird.

Für den Fall, daß der Benutzer des Sitz- oder Ruhemöbels aus irgendwelchen Gründen keinerlei Pendelbewegung wünscht, kann diese über eine Rastvorrichtung in an sich bekannter Weise ausgeschlossen werden.

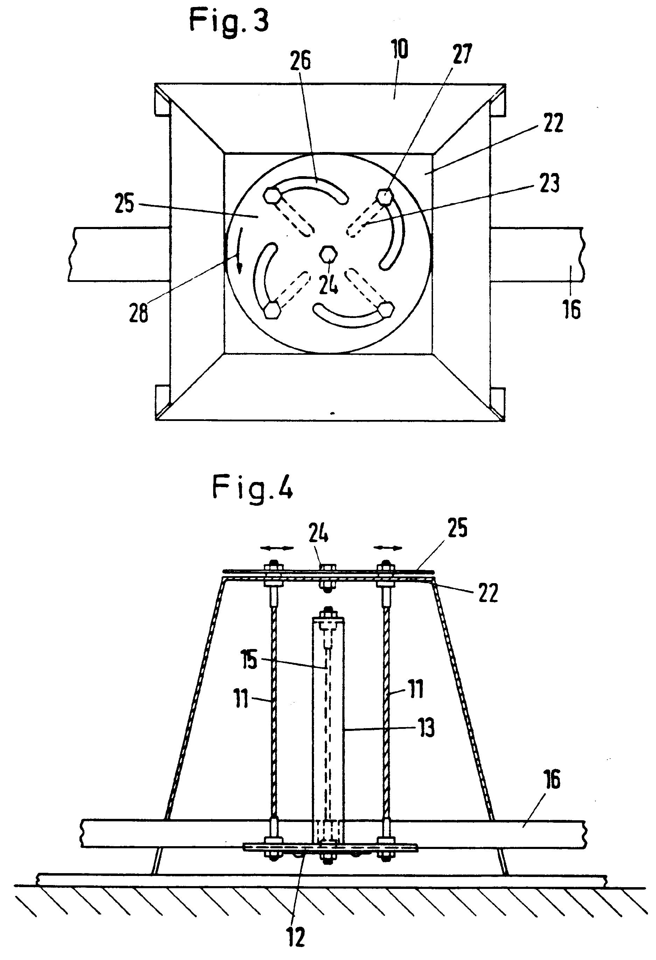

Bei der in Fig. 3 in der Draufsicht dargestellten Pendeleinrichtung ist die obere Deckplatte 22 des Gestells 10 mit Schlitzen 23 versehen, die radial in Richtung auf einen gemeinsamen Mittelpunkt 24 verlaufen. Auf der Deckplatte 22 ist eine um den Mittelpunkt 24 drehbare Scheibe 25 mit im wesentlichen spiralförmig verlaufenden Schlitzen 26 vorhanden. Die Seilpendel 11 sind über kopfförmige Aufhängepunkte 27, die auch die Gestalt von Schraubenmuttern haben können, derart aufgehängt, daß sie sowohl durch die radial verlaufenden Schlitze 23 der Deckplatte 22 des Gestells 10 als auch durch die im wesentlichen spiralförmig verlaufenden Schlitze 26 der um den Mittelpunkt 24 drehbaren Scheibe 25 hindurchtreten. Durch ein Verdrehen der Scheibe 25 in Richtung des Pfeils 28 verschieben sich die oberen Aufhängepunkte der Seilpendel 11 in Richtung auf den Mittelpunkt 24. Dadurch kann beispielsweise aus einem sich nach oben öffnenden Trapez aus den Seilpendeln 11 und der Brücke 12 ein Rechteck und bei weiterer Verstellung ein sieh nach oben verjüngendes Trapez entstehen. Es bietet sich somit eine kontinuierliche Verstellmöglichkeit für die aus den Seilpendeln 11 und der Brücke 12 gebildeten Trapez und damit eine kontinuierliche Einflußnahme auf das Schwingungsverhalten. Damit hat der Benutzer die Möglichkeit, das Schwingungsverhalten seinen jeweiligen Wünschen und Bedürfnissen stufenlos anzupassen. Bei einer parallelen Einstellung der Seilpendel 11 ergibt sich ein normales Schwingunsverhalten. Je nach Abweichung aus der Parallelstellung der Seilpendel 11 in der einen oder in der anderen Richtung tritt ein vom Normalfall abweichendes Schwingungsverhalten auf.

## Patentansprüche

1. Sitz- oder Ruhemöbel mit relativ zueinander beweglichem Ober- und Unterteil, wobei die Sitz- bzw. Ruhefläche auf dem Oberteil vorgesehen ist, das mittels unter ihm angeordneter und nach unten ragender Arme über mindestens zwei Satz funktionell in Reihe angeordnete Hängependel, die innerhalb eines Satzes gleiche, von Satz zu Satz jedoch gegebenenfalls auch unterschiedliche Länge aufweisen, an dem sich unmittelbar oder mittelbar auf dem Boden abstützenden Unterteil aufgehängt sind, wobei es sich bei den Hängependeln um auf geringe körpermotorische Impulse (z.B. Herz- und Atemfrequenz) reagierende Seilpendel handelt, dadurch gekennzeichnet, daß in der einen Stufe zwei spiegelbildlich zueinander und parallel zu ihrer Hauptrichtung angeordnete Brücken (12) vorhanden sind, die über Seilpendel (11, 11a) zumindest in Bezug auf ihre Hauptrichtung trapezförmig aufgehängt und über ein auf den erwähnten Brücken (12) fußendes galgenförmiges Gerüst (13) starr miteinander verbunden sind, wobei ein als Querbalken (14) des Gerüsts (13) fungierendes Teil seinerseits ein Seilpendel (15) der zweiten Stufe trägt, an dem das Oberteil (16) des Sitz- oder Ruhemöbels aufgehängt ist.

2. Möbel nach Anspruch 1, dadurch gekennzeichnet, daß das am galgenförmigen Gerüst (13) aufgehängte Seilpendel (15) eine geringfügig von den Pendeln (11, 11a) der ersten Stufe abweichende Pendellänge aufweist.

3. Möbel nach einem der Ansprüche 1 oder 2, dadurch gekennzeichnet, daß zumindest die Pendel einer Stufe (11, 11 a, 15) von Hülsen (20) mit darin angeordneten Schmiegungstrichtern (21) umgeben sind.

4. Möbel nach Anspruch 3, dadurch gekennzeichnet, daß die Schmiegungstrichter-(21) einen sich verjüngenden kreisförmigen lichten Querschnitt aufweisen.

5. Möbel nach Anspruch 3, dadurch gekennzeichnet, daß die Schmiegungstrichter (21) einen sich verjüngenden ovalen lichten Querschnitt aufweisen.

6. Möbel nach einem der Ansprüche 1 bis 5, dadurch gekennzeichnet, daß die oberen Aufhängungspunkte (27) zumindest einer Stufe der Seilpendel (11, 11 a, 15) einerseits in radial auf einen gemeinsamen Mittelpunkt (24) verlaufenden Schlitzen (23) und andererseits in etwa spiralig verlaufenden Schlitzen (26) einer um dieser Mittelpunkt (24) drehbaren Scheibe (25) verlaufen.

## Claims

1. Piece of furniture for sitting or resting on with relatively movable upper part and lower part, wherein the seating or resting surface is provided on the upper part, which is suspended by means of downwardly projecting arms, which are arranged thereunder, by way of at least two sets of suspension pendulums, which are functionally arranged in series and which within one set have the same length but optionally different lengths from set to set, at the lower part directly or indirectly supported on the base, wherein in the case of the suspension pendulums there are concerned cable pendulums responsive to small motor pulses of the body (for example heart and respiration rate), characterised thereby that present in the one stage are two bridges (12), which are arranged in mirror image relative to one another and parallel to their main direction, are suspended by way of cable pendulums (11, 11a) in trapezium shape at least with respect to their main direction and are rigidly connected together by way of a gallows-like frame (13) resting on the aforesaid bridges (12), wherein a part functioning as crossbeam (14) of the frame (13) in turn carries a cable pendulum (15) of the second stage, at which the upper part (16) of the piece of furniture for sitting or resting on is suspended.

2. Piece of furniture according to claim 1, characterised thereby that the cable pendulumn (15) suspended at the gallows-like frame (13) has a pendulum length slightly departing from the pendulums (11, 11a) of the first stage.

3. Piece of furniture according to one of claims 1 or 2, characterised thereby that at least the pendulums of one stage (11, 11a, 15) are enclosed by sleeves (2a) with embracing funnels (21) arranged therein.

4. Piece of furniture according to claim 3, characterised thereby that the embracing funnels (21) have a tapering, circular cross-section.

5. Piece of furniture according to claim 3, characterised thereby that the embracing funnels (21) have a tapering, oval clear cross-section.

6. Piece of furniture according to one of claims 1 to 5, characterised thereby that the upper suspension points (27) at least of one stage of the cable pendulum (11, 11a, 15) run on the one hand in slots (23), which extend radially to a common centre point (24), and on the other hand in approximately spirally extending slots (26) of a disc (25) rotatable about this centre point (24).

## Revendications

1. Siège ou meuble de repos comprenant une partie inférieure et une partie supérieure, mobiles l'une par rapport à l'autre, la surface de siège ou de repos étant prévue sur la partie supérieure qui est suspendue à la partie inférieure s'appuyant directement ou indirectement sur le sol, grâce à des bras disposés en dessous de ladite partie supérieure et saillant vers le bas, et, par l'intermédiaire d'au moins deux groupes de pendules suspendus, qui sont disposés fonctionnellement en rangées et qui sont constitués de pendules de câbles réagissant à de très faibles impulsions corporelles (par exemple la fréquence des battements du coeur ou de la respiration), les pendules d'un même groupe étant de longueur identique mais la longueur des pendules étant différente d'un groupe à l'autre, *caractérisé en ce que* il est prévu dans l'un des niveaux, deux ponts (12) disposés symétriquement et parallèlement à la direction principale, suspendus à des pendules de câbles (11), (11a) en formant avec eux des trapèzes dans le plan de la direction principale, reliés de manière rigide entre eux par un cadre (13) en forme de potence prenant appui sur lesdits ponts (12), la traverse (14) du cadre (13) en forme de potence portant un pendule de câble (15) du deuxième niveau, auquel est suspendue la partie supérieure (16) du siège ou meuble de repos.

2. Siège ou meuble selon la revendication 1 *caractérisé en ce que* la longueur du pendule de câble (15) suspendu au cadre (13) en forme de potence diffère légèrement de celle des pendules du premier niveau.

3. Siège ou meuble selon les revendications 1 ou 2 *caractérisé en ce que* les pendules d'au moins l'un des niveaux (11), (11a), (15) sont entourés par des douilles (20) dans lesquelles sont logés des entonnoirs osculateurs (21).

4. Siège ou meuble selon la revendication 3 *caractérisé en ce que* les entonnoirs osculateurs (21) présentent une section transversale de forme intérieure circulaire, diminuant progressivement.

5. Siège ou meuble selon la revendication 3 *caractérisé en ce que* les entonnoirs osculateurs (21) présentent une section transversale de forme intérieure ovale, diminuant progressivement.

6. Siège ou meuble selon les revendications 1 à 5 *caractérisé en ce que* les points de suspension supérieurs (27) des pendules de câbles (11), (11a) (15) d'au moins l'un des niveaux se déplacent, d'une part, dans des fentes radiales (23) orientées vers un centre commun (24) et, d'autre part, dans des fentes (26) disposées sensiblement en spirale, ménagées dans un disque (25) mobile autour du centre (24).
